(19) Europäisches Patentamt

European Patent Office

Office européen des brevets

(11) Publication number: **0 097 521**

**A1**

(12) **EUROPEAN PATENT APPLICATION**

(21) Application number: 83303551.2

(22) Date of filing: 20.06.83

(51) Int. Cl.³: **A 61 F 5/44**

(30) Priority: 22.06.82 ZA 824416

(43) Date of publication of application:
04.01.84 Bulletin 84/1

(84) Designated Contracting States:
AT BE CH DE FR GB IT LI NL SE

(71) Applicant: Melvill, Roger Laidman
6 Dalziel Road
Wynberg Cape Province(ZA)

(72) Inventor: Melvill, Roger Laidman
6 Dalziel Road
Wynberg Cape Province(ZA)

(74) Representative: Wood, Anthony Charles et al,
c/o MICHAEL BURNSIDE & PARTNERS 2 Serjeants' Inn
Fleet Street
London EC4Y 1HL(GB)

(54) Incontinence sheath applicator.

(57) An incontinence sheath applicator comprises a length of tube (10) which is open at each end and from the side of which extends a hollow spigot (12). A one-way valve (14,16) allows air to flow along the spigot from the tube but inhibits flow to the tube. A barrel with a plunger in it connects to the spigot and enables air to be drawn through the valve. The applicator is used to prepare a sheath (18) for attachment to the male organ by passing the sheath through the tube and turning both ends (20,22) of the sheath back over the ends of the tube. The space between the tube and the sheath is then evacuated by withdrawing air through the valve. This expands the sheath. A bellows or any other means can be used to withdraw air through the valve. The evacuating means (44) may be such that it is held in the position in which the space is evacuated, and then the one-way valve can be omitted. An example of this is a strong bellows which is allowed to expand to evacuate the space.

FIG. 1

EP 0 097 521 A1

THIS INVENTION relates to an incontinence sheath applicator.

Incontinent male patients in hospitals and old age homes are generally provided with an incontinence device overnight so that leaking urine flows to a drainage receptacle. Many method of ensuring that the sheath of the device stays in situ have been proposed and these are largely successful in operation. However, applicant is not aware of any applicator which has been developed to assist the nurse in actually applying the incontinence sheath. This task is not an easy one when the male organ is flaccid.

The object of the present invention is to provide an applicator which assists a nurse or other medical practitioner to attach the sheath of an incontinence device to a male patient.

According to one aspect of the present invention there is provided an incontinence sheath applicator comprising a length of tube which is open at both ends and which is capable of receiving an incontinence sheath, an outlet in the walling of the tube and adapted for connection to means for evacuating air from the tube, and a one-way valve for

- 3 -

inhibiting air flow through the inlet into the tube.

Preferably said outlet is in the form of a
hollow spigot formed with an internal seat, there
being a ball which co-operates  with the seat, the
ball and seat constituting the one-way valve.

According to another aspect of the present
invention there is provided an incontinence sheath
applicator comprising a tube which is open at both
ends and which is capable of receiving an incontinence
sheath, an outlet in the walling of the tube, and
means for evacuating air from the tube through said
outlet after both ends of the tube have been sealed by
an incontinence sheath passing through the tube and
for maintaining the evacuated condition until the seal
at at least one end of the tube is broken.

According to a further aspect of the present
invention there is provided, in combination, an
incontinence sheath applicator and an evacuating
means, said applicator comprising a tube which is open
at both ends and which is capable of receiving an
incontinence sheath, and a hollow spigot which
protrudes from the tube and leads to the evacuating
means, and the evacuating means comprising a barrel, a
plunger in the barrel and a latch for holding the
plunger in a withdrawn position.

According to still another aspect of the
present invention there is provided a method of
preparing an incontinence sheath for application to a
male patient, the sheath being in the form of a length
of flexible tubing and the method comprising feeding
the sheath through a tube, stretching each end of the tubing to increase its diameter and turning it back on itself so that it grips around a respective end of the tube to seal the ends of the tube, and drawing air from the space between the tube and the tubing so as to expand the sheath.

The sheath prepared as defined can be applied by inserting the male penis into the sheath through one end thereof, and releasing both ends of the tubing from the tube so that the tubing contacts onto the penis.

In accordance with one specific method the sheath is in the form of a length of tubing with a tapering connector at one end, and the method comprises feeding the length of tubing through the tube, stretching the free end of the length of tubing so as to increase its diameter and turning it back on itself so that it grips around one end of said tube to seal that end of the tube, stretching the tubing adjacent the connector so as to increase its diameter and turning it back on itself to form a pleat which seals the other end of the tube, and withdrawing air from the space between the tube and tubing so as to expand the length of tubing.

The method can further include the step of passing a ring over the connector to trap said pleat between the ring and and the tube.

The sheath can be applied by inserting the male penis into the sheath through said free end thereof, and releasing both ends of the sheath from the tube so that the tubing contracts onto the penis.

For a better understanding of the present invention reference will now be made, by way of example, to the accompanying drawing in which :-

Figure 1 is a section through an incontinence sheath applicator in accordance with the present invention, Figure 1 also showing part of an incontinence sheath, and

Figure 2 is a section illustrating a further incontinence sheath applicator together with a further incontinence sheath, and an air evacuator.

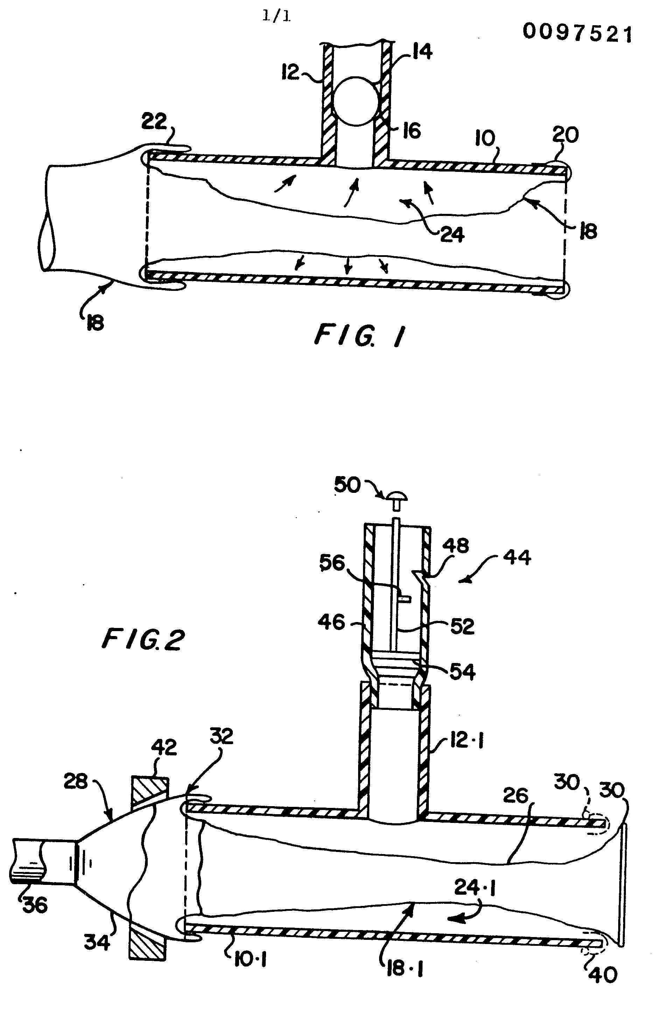

The applicator illustrated in Figure 1 comprises a length of tube 10 which is open at both ends. A hollow spigot 12 protrudes from the side wall of the tube 10 and, within the spigot 12, there is a ball 14. The ball 14 co-operates with a seat 16 and inhibits flow of air along the spigot 12 and into the tube 10. Suitable means (not shown) are provided for trapping the ball in the spigot 12. The ball valve illustrated can be replaced by any other suitable one-way valve such as a flap valve.

The incontinence sheath 18 is in the form of a length of soft tubing.

In use, the tubing constituting the sheath 18 is fed through the tube 10 from one end (the left hand end as illustrated) until a short portion of the free end of the tubing protrudes from the other end of the tube. This short portion is stretched to increase its diameter and then turned back on itself so that it tightly grips said other end of the tube 10. The portion which has been turned back on itself is designated 20 in Figure 1. It will be understood that, because of its elasticity, the turned back portion of the tubing grips the tube 10.

The part of the tubing constituting the
sheath 18 which is within said one end of the tube 10
is intussuscepted, that is, stretched outwardly to
increase its diameter and then formed with a
circumferentially extending pleat 22 so that it grips
around said one end of the tube 10. As will clearly
be seen from Figure 1, the rest of the sheath 18
extends away from said one end of the tube 10 (to the
left as illustrated in Figure 1).

There is now an annular space 24 between the
tube 10 and the sheath 18 which space is sealed at
both ends by the sheath. This annular space 24 is
then evacuated via the spigot 12. For example, a
conventional syringe comprising a barrel and a plunger
can be applied to the spigot 12 and the plunger
thereof withdrawn. Evacuating the space 24 causes the
sheath 18 to be drawn outwardly, as shown by the
arrows, against the inner cylindrical face of the tube
10. When the syringe is removed the ball 14 is
immediately urged by atmospheric pressure against the
seat 16 and inhibits airflow into the space 24.

The wide open sheath can then be applied to
the male patient by inserting his penis into said
other end of the tube and pushing the portion 20 off
the tube. The vacuum in the space 24 is thus relieved
and the sheath 18 contracts onto the penis. The free,
left hand end of the sheath 18 is then pulled while
the tube 10 is still gripped, thereby to pull out the
pleat 22. The tube 10 is then removed by displacing
it to the left as viewed in Figure 1 until it is
completely clear of the sheath 18.

It will be understood from the above that the applicator is evacuated by means of a syringe of the type in conventional use. It is, of course, possible to provide an applicator which includes its own evacuating means. Additionally, sheaths which are more complicated than the simple tubing described above are also in common use. An applicator including its own evacuator is shown in Figure 2 which also shows a different form of sheath. Where applicable, like references have been employed with the suffix .1. The tube 10.1 and spigot 12.1 are shown in section and the sheath 18.1 partly in section and partly in elevation. The spigot 12.1 does not include a one-way valve.

The sheath 18.1 comprises a length of soft tubing 26 heat sealed to a connector 28. The connector 28 is of thicker material than the tubing 26. A bead 30 is provided around the free end of the tubing 26. The connector 28, which is joined to the tubing 26 along the line 32, includes a hollow tapering portion 34, and a hollow tubular portion 36.

The evacuating structure of the applicator of Figure 2 is designated 44 and includes a generally cylindrical barrel 46 which tapers at one end to provide a portion which fits in the bore of the spigot 12.1. The side wall of the barrel 46 is indented to form an internal protuberance 48. For example a hot implement can be pressed against the outside of the barrel to indent it.

Within the barrel 46 there is a plunger 50 comprising a rod 52 and a piston 54. A latch 56 protrudes from the rod 52.

In use of the applicator of Figure 2, the sheath 18.1 is fed into the tube 10 from the left hand end thereof with the bead 30 leading. The length of the tube 10.1 with respect to the length of the sheath 18.1 is such that the bead 30 and a small portion of the tubing 26 protrude from the other end of the tube 10.1 before the portion 32 enters the tube 10.1. The portion of the tubing 26 adjacent the bead 30 is stretched outwardly and turned back on itself so as tightly to grip around said other end of the tube 10.1 as shown in dotted lines. The portion which is turned back on itself is designated 40.

At the other end of the tube 10.1, the tubing 26 is intussuscepted close to the line 32 so that it forms a pleat which grips the tube 10.1. To improve the sealing effect it is possible to provide a ring 42 the bore of which is tapered. When the ring 42 is pressed against the pleat, the sheath 18.1 is gripped between the end of the tube 10.1 and the ring 42 thus ensuring an airtight closure.

To evacuate the space 14.1, the plunger 50 is pulled upwardly from the position illustrated. By rotating the plunger 50, the latch 56 can be brought to a position in which it clears the internal protuberance 48. Thereafter, it can be further rotated to the position in which the latch 56 is immediately above the protuberance 48 which thus prevents downward movement of the plunger and maintains the low pressure condition in the space 24.1.

The sheath 18.1 is applied in the same manner as the sheath 18 of Figure 1. Pushing the portion 40 and the pleat off the tube 10.1 relieves the vacuum in the space 24.1 and causes the sheath to contract onto the penis of the male patient.

The structure 44 can be permanently connected to the spigot 12.1 or can be an air-tight push fit in the spigot. If it is an air-tight push fit, this facilitates packing and storage as the structure 44 then fits in the tube 10.1 when not in use. Another possible form of evacuator comprises a strong bellows including a clip which, after the bellows has been collapsed, holds it in this condition. After the sheath 18.1 has been attached to the tube 10.1, the bellows clip is released to allow the bellows to expand. The increase in volume of the bellows lowers the pressure in the space 24.1 sufficiently to cause the sheath 18.1 to expand. The bellows can be spring loaded internally to assist in holding it expanded.

Sheaths are available the connectors of which have an annular shoulder close to the junction between the connector and the tubing. If such shoulder, if necessary with the assistance of a ring such as ring 42, forms a sufficiently air-tight seal with the tube 10, 10.1, then it is not necessary to pleat the tubing adjacent the connector. To improve the seal, the respective end of the tube can be shaped, such as tapered, to conform with the shape of the connector.

0097521

- 10 -

CLAIMS:

1. An incontinence sheath applicator comprising a tube which is open at both ends and which is capable of receiving an incontinence sheath, an outlet in the walling of the tube and adapted for connection to means for evacuating air from the tube, and a one-way valve for inhibiting air flow through the inlet into the tube.

2. An applicator according to claim 1, in which said outlet is in the form of a hollow spigot formed with an internal seat, there being a ball which co-operates with the seat, the ball and seat constituting the one-way valve.

3. An incontinence sheath applicator comprising a tube which is open at both ends and which is capable of receiving an incontinence sheath, an outlet in the walling of the tube, and means for evacuating air from the tube through said outlet after both ends of the tube have been sealed by an incontinence sheath passing through the tube and for maintaining the evacuated condition until the seal at at least one end of the tube is broken.

4. In combination, an incontinence sheath applicator and an evacuating means, said applicator comprising a tube which is open at both ends and which is capable of receiving an incontinence sheath, and a hollow spigot which protrudes from the tube and leads to the evacuating means, and the evacuating means comprising a barrel, a plunger in the barrel and a latch for holding the plunger in a withdrawn position.

5.      A method of preparing an incontinence sheath for application to a male patient, the sheath being in the form of a length of flexible tubing and the method comprising feeding the sheath through a tube, stretching each end of the tubing to increase its diameter and turning it back on itself so that it grips around a respective end of the tube to seal the ends of the tube, and drawing air from the space between the tube and tubing so as to expand the sheath.

6.      A method of applying a sheath prepared by the method claimed in claim 5, which comprises inserting the male penis into the sheath through one end thereof, and releasing both ends of the tubing from the tube so that the tubing contracts onto the penis.

7.      A method according to claim 5, in which the sheath is in the form of a length of tubing with a tapering connector at one end, and the method comprises feeding the length of tubing through the tube, stretching the free end of the length of tubing so as to increase its diameter and turning it back on itself so that it grips around one end of said tube to seal that end of the tube, stretching the tubing adjacent the connector so as to increase its diameter and turning it back on itself to form a pleat which seals the other end of the tube, and withdrawing air from the space between the tube and tubing so as to expand the length of tubing.

0097521

- 12 -

8.       A method as claimed in claim 7, and including the step of passing a ring over the connector to trap said pleat between the ring and the tube.

9.       A method of applying a sheath prepared by the method claimed in claim 7  or 8, which comprises inserting the male penis into the sheath through said free end thereof, and releasing both ends of the sheath from the tube so that the tubing contracts onto the penis.

FIG. 1

FIG. 2

# PARTIAL EUROPEAN SEARCH REPORT

which under Rule 45 of the European Patent Convention shall be considered, for the purposes of subsequent proceedings. as the European search report

**European Patent Office**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | WO - A - 81/03609 (L. BRENDLING) | | A 61 F 5/44 |
| | * Claim 10; page 6, line 31 - page 7, line 4 * | 1,3 | |
| | * Claim 8 * | 5 | |
| | * Claim 5 * | 7 | |
| | -- | | |
| A | US - A - 3 511 241 (J.W. LEE) | | |
| | ---- | | |

|  |
|---|
| TECHNICAL FIELDS SEARCHED (Int. Cl. ³) |
| A 61 F<br>A 61 B |

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-5,7-8

Claims searched incompletely:

Claims not searched: 6,9

Reason for the limitation of the search:

Method for treatment of the human or animal body by surgery or therapy (see article 52 (4) of the European Patent Convention).

| Place of search | Date of completion of the search | Examiner |
|---|---|---|
| The Hague | 14-09-1983 | KNAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document